Europäisches Patentamt

European Patent Office

Office européen des brevets

(11) Publication number: **0 479 158 A1**

# EUROPEAN PATENT APPLICATION

(21) Application number: **91116550.4**

(22) Date of filing: **27.09.91**

(51) Int. Cl.⁵: **C12Q 1/68**, //C12Q1/70

(30) Priority: **29.09.90 JP 262457/90**

(43) Date of publication of application:
**08.04.92 Bulletin 92/15**

(84) Designated Contracting States:
**BE CH DE FR GB IT LI NL SE**

(71) Applicant: **SHIMADZU CORPORATION**
**1 Kuwabara-cho Nishinokyo Nakakyo-ku**
**Kyoto 604(JP)**

(72) Inventor: **O-Hashi, Tetsuo**
**18, Shunei-cho, Saiin Ukyo-ku**
**Kyoto-shi, Kyoto-fu(JP)**

(74) Representative: **Vossius & Partner**
**Siebertstrasse 4 P.O. Box 86 07 67**
**W-8000 München 86(DE)**

(54) **Reagent for RNA detection and RNA detection method using it.**

(57) The present invention is directed to a reagent for RNA detection comprising at least both reverse transcriptase and thermostable DNA polymerase, and a RNA detection method using the reagent.

The present invention makes it possible to perform the reverse transcription reaction in the same reaction solution as with the PCR reaction. This obviates the necessity of transfer of the reaction solution and replacement of the buffer system, thus making it possible to produce an amplified DNA fragment solely by controlling the reaction temperature, whereby the detecting operation is significantly simplified and the time required before detection is shortened.

EP 0 479 158 A1

The present invention relates to a method of detecting RNA by amplifying a particular base sequence possessed thereby.

Gene amplification, represented by the PCR technique, serves excellently for the purpose of specifically detecting a nucleic acid having a particular base sequence in a short time. Gene amplification techniques include the polymerase chain reaction technique [hereinafter referred to as the PCR technique; Science, 230, 1350 (1985)], which was developed by Saiki et al. However, since the nucleic acid used as a template for gene amplification by the PCR technique must be supplied in the form of DNA, RNA samples, which are the subject of detection in the present invention, cannot undergo direct gene amplification. It is therefore necessary to transfer the genetic information of RNA to DNA to detect the RNA by the PCR technique. Reverse transcriptase is used to transfer the genetic information from RNA to DNA. At laboratory levels, a reverse transcriptase buffer is used to carry out the reaction in a reactor different from the reactor for the PCR reaction for gene amplification; therefore, the reagent must be re-prepared to change the composition of the reaction solution or transferred from a reactor to another one for the PCR reaction which follows. These procedures are troublesome and take a great deal of time, and there has been no means other than manual operation. Particularly, changing the composition of the reaction solution is a troublesome and time-consuming operation involving ordinary DNA purification processes such as phenol treatment, chloroform treatment and ethanol precipitation.

It is an object of the present invention to provide a reagent for RNA detection which permits simple detection of RNA by the PCR technique and an RNA detection method using it.

It is necessary to establish an environment in which reverse transcriptase and thermostable DNA polymerase (Taq DNA polymerase), which function in the reverse transcription reaction and the PCR reaction, respectively, both remain active and in which their enzyme activities can be artificially controlled. In addition, to facilitate the switching of the two reactions, reverse transcriptase and Taq DNA polymerase are allowed to be concurrently present in the reaction solution and the reactions are switched to each other solely by controlling the temperature.

The reverse transcription reaction and the gene amplification reaction with DNA polymerase are both primer elongation reactions, and the enzymes for the respective reactions utilize four kinds of deoxynucleotide triphosphate (dATP, dGTP, dCTP, TTP) as substrates. Also, the two enzymes are similar to each other with respect to optimum hydrogen ion concentration for maximum activity and optimum concentration of monovalent or divalent cation; therefore, they permit the use of a reaction buffer in common. The hydrogen ion concentration is in the pH range of 8.0 to 9.0. As for the cation concentration, it is preferably 20 to 80 mM for a monovalent cation of $K^+$ or 1.5 to 3 mM for a divalent cation of $Mg^{2+}$. It is the optimum temperature that significantly differentiates the two enzymes from each other; the optimum temperature of reverse transcriptase is 37 to 42°C, while that of Taq DNA polymerase is 70 to 75°C. Utilizing this difference makes it possible to switch from reverse transcription reaction to gene amplification reaction solely by controlling the reaction chamber temperature. In fact, the gene amplification reaction comprises thermal denaturation, annealing and primer chain elongation; reverse transcriptase is deactivated by the temperature for thermal denaturation (90 to 96°C). Thus, the reverse transcriptase activity which became unnecessary upon switching to the gene amplification reaction is eliminated. On the other hand, thermostable DNA polymerase does not undergo irreversible deactivation during reverse transcription reaction, though its activity decreases because the reverse transcription reaction temperature is considerably lower than the optimum temperature. It exhibits DNA polymerase activity upon switching to the gene amplification reaction. The entire course of these reactions proceeds in the same reactor without involving sample dispensing or reagent addition during their progress, and they are carried out solely by heating and cooling from outside the reactor. Therefore, no manual operation is needed, as long as a constant temperature chamber is available in which temperature and time can be controlled on an arbitrary program by means of a computer.

Figure 1 is a photograph of the fluorescence pattern of the nucleic acid bands obtained in Example 1.

Figure 2 is a photograph of the fluorescence pattern of the nucleic acid bands obtained in Example 2.

The process flow from reverse transcription reaction to amplification reaction in the present invention is described below. If the sample contains an RNA molecule having a sequence to be detected, reverse transcriptase acts to synthesize a complementary DNA chain from the RNA as a template in the presence of an oligonucleotide primer. The oligomer which functions as a primer in this reaction may be an oligomer having a sequence which anneals to a particular sequence which serves as a primer in the subsequent gene amplification by the PCR reaction, or a hexameric or similar random primer which causes priming at an uncertain site on the RNA chain may be used for reverse transcription reaction. Since the purpose of the reverse transcription reaction is to form a complementary DNA which serves as a template for the region to be amplified by the PCR reaction, the reverse transcription reaction must be controlled to ensure the obtainment of a minimum necessary number of copies of the target sequence detectable in the amplification reaction. Reverse transcription reaction in the presence of excess reverse transcriptase should be avoided, since it inhibits the subsequent amplification reaction with Taq polymerase. After completion of the reverse transcription reaction, the thermal denaturation process is carried out as the first step of the amplification reaction. In this process, the reaction system temperature is raised to 94°C so that a single-stranded DNA is newly formed to prepare for annealing of the primer to be used in the amplification reaction. At the same time, the reverse transcriptase is deactivated, while the thermostable DNA polymerase alone remains active. Next, the reaction system temperature is lowered (37 to 65°C) to initiate the primer annealing process. In this process, a particular base sequence on the newly formed DNA and the primer hybridize to form a double-stranded hybrid in such manner that both ends of the amplification region are sandwiched thereby. Then, the temperature is raised to the optimum activity temperature (72°C) for Taq polymerase to initiate the DNA chain elongation reaction from the primer. These three processes are repeated in this order, whereby the amplification reaction proceeds.

To prevent the sample RNA from being decomposed by the ribonuclease present as an impurity substance in the sample, it is desirable to previously add a ribonuclease inhibitor to the reaction solution. Examples of ribonuclease inhibitors include vanadyl ribonucleoside complex, diethyl pyrocarbonate and the human placenta derived ribonuclease inhibitor (RNasin). It is recommended to use RNasin, since vanadyl ribonucleoside complex and diethyl pyrocarbonate both inhibit the enzyme activity of Taq polymerase.

Also, although the sample is preferably a purified RNA, the cell itself or the virus particle itself may be added directly to the reaction solution to detect a particular RNA in the cell or viral genomic RNA. In this case, it is preferable to previously add nonionic surfactants such as Nonidet P-40 and Tween 20 to the reaction solution to facilitate the release of RNA.

The present invention makes it possible to perform the reverse transcription reaction, which is necessary to detect RNA by gene amplification, in the same reaction solution as with the PCR reaction. This obviates the necessity of transfer of the reaction solution and replacement of the buffer system, thus making it possible to produce an amplified DNA fragment, the end product, solely by heating and cooling the reactor, whereby the detecting operation is significantly simplified and the time required before detection is shortened. Particularly, since the detection is effected solely by controlling the reaction temperature alone on a time basis, it is easy to apply the present invention to automated equipment which controls the temperature on the basis of a previously input program using a computer. Also, the invention is favorable for treatment of large amounts of samples, since it is expected to fairly simplify the sample pretreatment; for example, detection is possible even when virus particles are directly added. Thus, the present invention is particularly effective when it is used for clinical examinations for detection of RNA viruses etc.

## Examples

## Example 1

A base sequence of 20 base pairs having the following nucleotide sequence [contained in the gene which codes for the F protein of Sendai virus (Miura, N. et al., Gene, 38, 271-274 (1985))] was synthesized, using an automated DNA synthesizer.

5'-GATAGCTGGATCCCACGAAT-3'     (SEQ ID NO:1)
3'-CCGAGACTATTGACAGTGGT-5'     (SEQ ID NO:2)

The size of the DNA fragment resulting from amplification by the above primer under the PCR reaction is 173 base pairs. The sample was prepared from a cell culture supernatant containing Sendai virus by the method of Cantel et al. [Cantel, K. et al., Method in Enzymology, 78, 299 (1981)]. RNasin (final concentration = 400 u/ml) was added and then SDS (final concentration = 1%) was added and mixed. Next, an equal volume of a 1:1 phenol/chloroform mixture was added. After stirring, centrifugation was carried out at 5000 x g for 10 minutes. After completion of the centrifugation, the supernatant was recovered, and 2 parts by volume of 7.5 M ammonium acetate and 2.5 parts by volume of cold ethanol were added. The mixture was kept standing at -20°C for 2 hours and then centrifuged at 12000 x g for 15 minutes, and the precipitate was recovered to yield a purified RNA of Sendai virus. This RNA was used to prepare a reaction solution as follows.

50 mM KCl, 10 mM Tris-HCl (pH 9.0), 1.5 mM $MgCl_2$, 0.01% (w/v) gelatin, 0.4 mM dATP, 0.4 mM dGTP, 0.4 mM dCTP, 0.4 mM TTP, 20 pM of each of the above oligonucleotides, 40 units of RNasin (Toyobo Co., Ltd.), 190 pM random hexamer (Pharmacia), 200 units of M-MLV Reverse Transcriptase (Bethesda Research Laboratories Inc.), 2.5 units of Taq DNA polymerase (Perkin Elmer Cetus) and the above-mentioned RNA were added to reach a total volume of 30 $\mu$l. The amount of RNA added to the reaction system was respectively adjusted so that $10^8$, $10^6$, $10^4$ , $10^2$ or 0 copy of the genome of Sendai virus would be obtained. For a control experiment, another reaction solution was prepared in the same manner as above except that M-MLV Reverse Transcriptase alone was eliminated from the above-mentioned composition of the reaction solution. The RNA content in this standard preparation was determined from the UV absorption at a wavelength of 260 nm and converted to the number of copies of the viral genome.

This solution was heated at 42°C for 40 minutes and subjected to reverse transcription reaction by the action of M-MLV Reverse Transcriptase, followed by 35 cycles of amplification using the repeat program of 94°C for 1 minute, 50°C for 1 minute and 72°C for 1 minute. These heating and cooling procedures, including the reverse transcription reaction, were all performed in a program incubator (DNA Thermal Cycler, produced by Perkin Elmer Cetus).

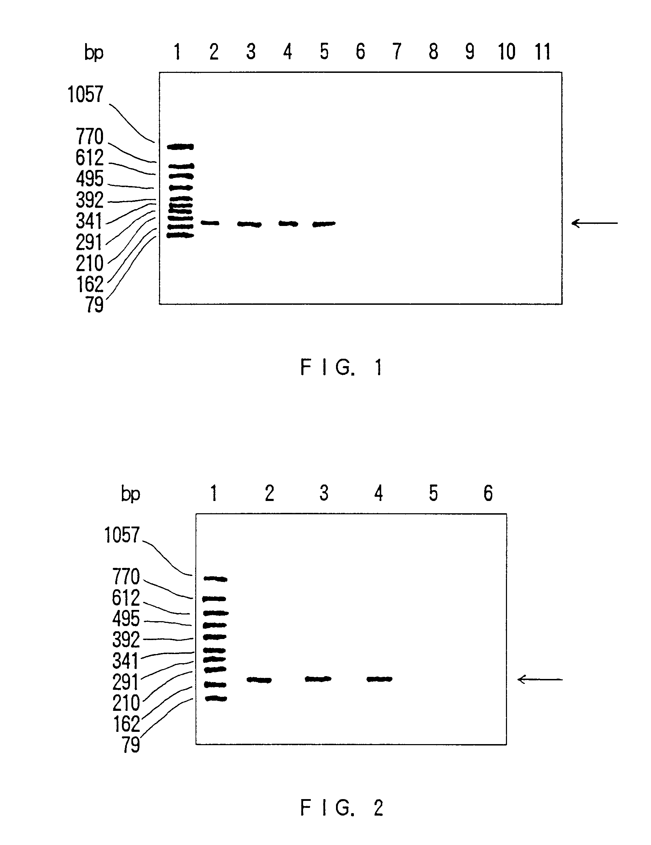

A 10 $\mu$l aliquot of the reaction solution was added to 3% agarose gel in a 40 mM Tris-acetate buffer containing 2 mM EDTA and 0.5 $\mu$g/ml ethidium bromide. After the gel was electrophoresed at 12 V/cm for 20 minutes, the UV fluorescence pattern of the resulting nucleic acid bands was photographed, using a Polaroid camera (see Figure 1). In Figure 1, lane 1 shows the bands from the standard molecular weight marker; lane 2 shows the band obtained in the presence of $10^8$ molecules of RNA of Sendai virus per reaction system; lanes 3 through 6 show the bands obtained in the presence of $10^6$, $10^4$, $10^2$ and 0 molecule of RNA of Sendai virus, respectively; lanes 7 through 11 show the results of the control experiment, in which the amount of RNA of Sendai virus added was $10^8$ molecules for lane 7, $10^6$ molecules for lane 8, $10^4$ molecules for lane 9, $10^2$ molecules for lane 10 and 0 molecule for lane 11.

The migrational positions of the detected bands were compared with those from the molecular weight marker ($\phi$x174/HincII) on lane 1 to estimate the length of the base pair sequence. The estimation agreed with the theoretical length of 173 base pairs, demonstrating that the gene which codes for the F protein of Sendai virus was successfully correctly amplified (indicated by arrow in Figure 1). Also, the detection limit was found to be $10^2$ molecules on lane 5, from which it is evident that the present invention offers a detection sensitivity which is comparable to that obtained by the ordinary reaction in which DNA is detected directly by the PCR technique. From the reaction solution containing no M-MLV Reverse Transcriptase, no amplification band appeared. This finding suggests that no gene amplification occurs unless the RNA of Sendai virus is converted to DNA by reverse transcription (lanes 7 through 11).

Example 2

An experiment was made in the same manner as in Example 1 except that Sendai virus particles, in place of the standard preparation of RNA, were added directly to the reaction system. The Sendai virus particles used were obtained by inoculating the seed virus to a 10-day incubated chicken egg and purifying the proliferated virus by ultracentrifugation in accordance with the above-mentioned method of Cantel et al. This sample was used to prepare a reaction solution as follows.

50 mM KCl, 10 mM Tris-HCl (pH 9.0), 1.5 mM MgCl$_2$, 0.01% (w/v) gelatin, 0.4 mM dATP, 0.4 mM dGTP, 0.4 mM dCTP, 0.4 mM TTP, 20 pM of each of the above oligonucleotides, 0.05% Tween 20, 0.05% Nonidet P-40, 40 units of RNasin (Toyobo Co., Ltd.), 200 units of M-MLV Reverse Transcriptase (Bethesda Research Laboratories Inc.), 2.5 units of Taq DNA polymerase (Perkin Elmer Cetus) and the above-mentioned RNA were added to reach a total volume of 30 $\mu$l. The amount of RNA added to the reaction system was respectively adjusted so that $10^8$, $10^6$, $10^4$, $10^2$ or 0 copy of the genome of Sendai virus would be obtained. As a control, another reaction solution was prepared in the same manner as above except that M-MLV Reverse Transcriptase alone was eliminated from the above composition of the reaction solution. The number of virus particles in this standard preparation was calculated from the amount of RNA extracted from the same standard preparation by the method described in Example 1.

This solution was heated at 42°C for 40 minutes and subjected to reverse transcription reaction by the action of M-MLV Reverse Transcriptase, followed by 35 cycles of amplification using the repeat program of 94°C for 1 minute, 50°C for 1 minute and 72°C for 1 minute. These heating and cooling procedures, including the reverse transcription reaction, were all performed in a program incubator (DNA Thermal Cycler, produced by Perkin Elmer Cetus).

A 10 $\mu$l aliquot of the reaction solution was added to 3% agarose gel in a 40 mM Tris-acetate buffer containing 2 mM EDTA and 0.5 $\mu$g/ml ethidium bromide. After the gel was electrophoresed at 12 V/cm for 20 minutes, the UV fluorescence pattern of the resulting nucleic acid bands was photographed, using a Polaroid camera (see Figure 2).

Lane 1 shows the bands from the standard molecular weight marker ($\phi$x174/HincII); lane 2 shows the band obtained in the presence of $3 \times 10^7$ molecules of RNA of Sendai virus per reaction system; lanes 3 through 6 show the bands obtained in the presence of $3 \times 10^5$, $3 \times 10^3$, 300 and 0 molecule of RNA of Sendai virus, respectively.

As a result, it was found that virus particles could be detected at a level of $3 \times 10^3$ particles, since the band indicated by arrow on lane 3 of Figure 2 could be found, though the detection sensitivity was lower in comparison with the purified RNA used in Example 1. Since the RNA in virus particles is enveloped by protein, double membranes of lipid and other structures, the primer can not gain direct access thereto and the reverse transcription reaction can not take place. However, the reaction solution used in Example 2 contained surfactants Tween 20 and Nonidet P-40, which are considered to effectively act to release the RNA in the virus particles in a form which permits its reaction.

SEQUENCE LISTING

SEQ ID NO:1

SEQUENCE LENGTH: 20 bases

SEQUENCE TYPE: nucleic acid

STRANDEDNESS: single

TOPOLOGY: linear

MOLECULE TYPE: genomic DNA

HYPOTHETICAL: No

ANTI-SENSE: No

ORIGINAL SOURCE:

    ORGANISM: Sendai virus

FEATURE:

    IDENTIFICATION METHOD: S

SEQUENCE

GATAGCTGGA TCCCACGAAT           20

SEQ ID NO:2

SEQUENCE LENGTH: 20 bases

SEQUENCE TYPE: nucleic acid

STRANDEDNESS: single

TOPOLOGY: linear

MOLECULE TYPE: genomic DNA

HYPOTHETICAL: No

ANTI-SENSE: No

ORIGINAL SOURCE:

    ORGANISM: Sendai virus

```
FEATURE:

     IDENTIFICATION METHOD: S

SEQUENCE

CCGAGACTAT TGACAGTGGT                                    20
```

**Claims**

1. A reagent for RNA detection comprising at least both reverse transcriptase and thermostable DNA polymerase.

2. A RNA detection method using a reagent containing primers, dATP, dCTP, dGTP, TTP, reverse transcriptase and thermostable DNA polymerase comprising:
   (a) synthesizing a comlementary DNA chain from a RNA in a sample by a reverse transcription reaction;
   (b) amplifying said DNA by PCR technique using a thermostable DNA polymerase in the reaction solution obtained in the process (a); and
   (c) detecting the amplified DNA.

3. A RNA detection method according to claim 2, wherein said RNA in a sample is purified.

F I G. 1

F I G. 2

European
Patent Office

EUROPEAN SEARCH
REPORT

Application Number

EP 91 11 6550

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF CLINICAL MICROBIOLOGY vol. 28, no. 2, February 1990, pages 276 - 282; V.GOUVEA ET AL.: 'Polymerase chain reaction amplification and typing of rotavirus nucleic acid from stool specimens' * abstract * * page 277, right column, line 25 - page 278, left column, line 29 * * | 1-3 | C 12 Q 1/68 // C 12 Q 1/70 |
| Y | ONCOGENE vol. 4, 1989, pages 1149 - 1151; I.K.HEWLETT ET AL.: 'Co-amplification of multiple regions of the HIV-1 genome by the polymerase chain reaction: potential use in multiple diagnosis' * page 1149, right column, line 15 - line 43 * * | 1-3 | |
| Y | EP-A-0 290 144  (CITY OF HOPE) * column 4, line 1 - line 42 * * | 1-3 | |
| A | WO-A-8 810 315  (SISKA DIAGNOSTICS) * page 26, line 17 - line 30 * * claims 22,25,30,49 * * | 1 | |
| A | CLINICAL AND EXPERIMENTAL PHARMACOLOGY vol. 18, no. 5, May 1991, pages 357 - 362; YI-KUN LOU ET AL.: 'Renin gene expression in various tissues determined by single-step polymerase chain reaction' * abstract * * page 358, left column, line 36 - page 359, left column, line 4 * EP 91116550030 * | 1 | TECHNICAL FIELDS SEARCHED (Int. Cl.5)  C 12 Q |

The present search report has been drawn up for all claims

| Place of search | Date of completion of search | Examiner |
|---|---|---|
| The Hague | 04 November 91 | LUZZATTO E.R.P.G.A. |

CATEGORY OF CITED DOCUMENTS

X : particularly relevant if taken alone
Y : particularly relevant if combined with another
    document of the same catagory
A : technological background
O : non-written disclosure
P : intermediate document
T : theory or principle underlying the invention

E : earlier patent document, but published on, or after
    the filing date
D : document cited in the application
L : document cited for other reasons

&: member of the same patent family, corresponding
    document